# EUROPEAN PATENT APPLICATION

(11) **EP 3 854 879 A1**
(43) Date of publication of application: **28.07.2021**
(21) Application number: 19863931.2
(22) Date of filing: 20.09.2019
(51) Int. Cl.: C12N 15/867, C12N 5/10

(54) **METHOD FOR ENHANCING LENTIVIRUS VECTOR PRODUCTION**

(30) Priority: 20.09.2018 JP 2018176230
(71) Applicant: National University Corporation Tokyo Medical and Dental University, Tokyo 113-8510 (JP)
(72) Inventor: YAMAOKA, Shoji, Tokyo 113-8510 (JP); SUZUKI, Naoto, Tokyo 113-8510 (JP); YOSHIDA, Takeshi, Tokyo 113-8510 (JP)
(74) Representative: J A Kemp LLP
(86) International application number: PCT/JP2019/036929
(87) International publication number: WO 2020/059848

(57) **Abstract**

The present invention provides a method and a reagent for increasing lentiviral vector production in 293T cells. When the 293T cells are cotransfected with a packaging mix comprising a plurality of plasmids that comprise genes encoding proteins essential for lentiviral particle formation and a plasmid that comprises a gene transcribed into RNA to be incorporated into lentiviral particles containing a transgene to be expressed in target cells according to the method for producing a lentiviral vector, HTLV-1 Tax, HIV-1 Tat, or NF-κB RelA are coexpressed in the 293T cells, so as to increase the amount of lentiviral vector production.

SEQUENCE LISTING

<110>  National University Corporation Tokyo Medical and Dental University

<120>  A method for enhacing lentiviral vector production

<130>  PH-8070-PCT

<150>  JP 2018-176230
<151>  2018-09-20

<160>  4

<170>  PatentIn version 3.5

<210>  1
<211>  26
<212>  DNA
<213>  Artificial

<220>
<223>  Primer

<400>  1
ccggatccgc caccatggct tccaag                                          26


<210>  2
<211>  27
<212>  DNA
<213>  Artificial

<220>
<223>  Primer

<400>  2
aactcgagtt agacgttgat cctggcg                                         27


<210>  3
<211>  41
<212>  DNA
<213>  Artificial

<220>
<223>  Primer

<400>  3
gctctagagc tagcatggct tcgtacccct gccatcaaca c                         41


<210>  4
<211>  31
<212>  DNA
<213>  Artificial

<220>
<223>  Primer

<400>  4
cgcggatcct cagttagcct cccccatctc c                                    31

## Description

### [Technical Field]

The present invention relates to a method for efficiently producing a lentiviral vector.

### [Background Art]

A lentiviral vector derived from human immunodeficiency virus type 1 (HIV-1) is a valuable tool for transducing a foreign gene into a dividing cell and a non-dividing cell both *in vitro* and *in vivo.* Safety and usefulness of the lentiviral vector had been improved by various techniques (Non-Patent Document 1). The G glycoprotein of vesicular stomatitis virus (VSV-G)-pseudotyped lentiviral vector is produced under the control of the human Cytomegalovirus (CMV) immediate early promoter in HEK293T cells and it is capable of mediating very efficient transduction into an extensive range of cells (Non-Patent Document 2). Recent clinical application of the lentiviral vector targeting hematopoietic stem cells and T cells has achieved a significant success (Non-Patent Document 3). *In vivo* experiments and transduction into a non-dividing cell often require the use of a lentiviral vector having a high titer. To this end, it is necessary to produce a lentiviral vector via large-scale culture, concentrate the vector via ultracentrifugation, and purify the resultant via chromatography and ultrafiltration (Non-Patent Document 4). At present, such procedure is significantly disadvantageous due to the high cost incurred by vector production involving the use of a large quantity of plasmids, cell culture materials, and apparatuses for concentration and purification. While a great amount of energy had been expended aimed at improvement in the procedure for concentration and purification of the lentiviral vector from the culture supernatant, the number of reports made on a simple method for increasing vector production is relatively small. It was demonstrated that viral vector production would be increased by blocking the innate immune responses in cells (Non-Patent Document 5) and that lentiviral vector production would be increased by an enhanced transfection efficiency resulting from stable expression of the immunoreactive protein Siglec-9 (Non-Patent Document 6). Other researchers had conducted studies for increasing the lentiviral vector titer with the addition of caffeine (Non-Patent Document 7) or sodium butyrate (Non-Patent Document 8) to the culture medium. However, a further improvement is required to produce a lentiviral vector having a high titer.

### [Prior Art Documents]

### [Non-Patent Documents]

[Non-Patent Document 1] Matrai et al., Molecular therapy: The Journal of the American Society of Gene Therapy 18, 477-490, doi:10.1038/mt.2009.319, 2010
[Non-Patent Document 2] Burns, J. C. et al., Proceedings of the National Academy of Sciences of the United States of America 90, 8033-8037, 1993
[Non-Patent Document 3] Dunbar, C.E. et al., Science 359, doi:10.1126/science.aan4672, 2018
[Non-Patent Document 4] McCarron, A. et al., Journal of Biotechnology 240, 23-30, doi:10.1016/j.jbiotec.2016.10.016, 2016
[Non-Patent Document 5] de Vries et al., Gene therapy 15, 545-552, doi:10.1038/gt.2008.12, 2008
[Non-Patent Document 6] Shoji, T. et al., Cytotechnology 67, 593-600, doi:10.1007/s10616-013-9679-7, 2015
[Non-Patent Document 7] Ellis, B. L. et al., Human Gene Therapy 22, 93-100, doi:10.1089/hum.2010.068, 2011
[Non-Patent Document 8] Ansorge. S. et al., The Journal of Gene Medicine 11, 868-876, doi:10.1002/jgm.1370, 2009

### [Summary of the Invention]

### [Objects to Be Attained by the Invention]

A lentiviral vector is a valuable tool for delivery of a foreign gene for stable expression in cells. Development of a technique for purifying a lentiviral vector from a lentiviral vector-containing medium made a great progress. However, a method for increasing lentiviral vector production from producer cells has not been sufficiently studied.

The present invention provides a method and a reagent for increasing lentiviral vector production in 293T cells.

### [Means for Attaining the Objects]

Human T cell leukemia virus type 1 (HTLV-1) Tax protein (hereafter, referred to as "HTLV-1 Tax" or "Tax") is a trans-acting proteins that accelerates HTLV-1 LTR-driven transcription and activate cell transcription factors including the cAMP-response element-binding protein (CREB)/activating transcription factor (ATF), the intranuclear factor-B (NF-κB), and the activator protein-1 (AP-1) 1. The present inventors discovered that HTLV-1 Tax would increase the CMV promoter-driven gene expression in HEK293T cells and made use of the finding for lentiviral vector production.

The present inventors discovered that Gag expression in producer cells and lentiviral vector particle release from the producer cells would be enhanced to a significant extent upon simultaneous expression of a small amount of Tax protein and that transduction efficiency would be enhanced by over 10 times. This has led to the completion of the present invention comprising producing a lentiviral vector having a high titer through promoter activation in the producer cells.

Specifically, the present invention is as described below.
[1] A method for producing a lentiviral vector that enables substantial increase in lentiviral vector production, when cotransfecting 293T cells with a packaging mix comprising a plurality of plasmids that comprise genes encoding proteins essential for lentiviral particle formation and a plasmid that comprises a gene transcribed into RNA to be incorporated into lentiviral particles containing a transgene to be expressed in target cells, by simultaneously expressing factors which can activate promoters of transfected genes.
[2] The method for producing a lentiviral vector according to [1], wherein the factor activating a promoter is a factor activating a CMV promoter.
[3] The method according to [1] or [2], wherein the factor activating a promoter is one or more factors selected from the group consisting of HTLV-1 Tax, HIV-1 Tat, NF-κB RelA, AP-1, and CREB/ATF.
[4] The method for producing a lentiviral vector according to any of [1] to [3], which comprises, when cotransfecting 293T cells with a packaging mix comprising a plurality of plasmids that comprise genes encoding proteins essential for lentiviral particle formation and a plasmid that comprises a gene transcribed into RNA to be incorporated into lentiviral particles containing a transgene to be expressed in target cells, by simultaneously expressing HTLV-1 Tax or NF-κB RelA which can activate promoters of transfected genes in the 293T cells.
[5] The method for producing a lentiviral vector according to any of [1] to [3], which comprises, when cotransfecting 293T cells with a packaging mix comprising a plurality of plasmids that comprise genes encoding proteins essential for lentiviral particle formation and a plasmid that comprises a gene transcribed into RNA to be incorporated into lentiviral particles containing a transgene to be expressed in target cells, by simultaneously expressing 2 types of factors selected from the group consisting of HTLV-1 Tax, HIV-1 Tat, and NF-κB RelA or 3 types of factors HTLV-1 Tax, HIV-1 Tat, and NF-κB RelA which can activate promoters of transfected genes in the 293T cells.
[6] The method for producing a lentiviral vector according to [4] or [5], wherein the plurality of plasmids constituting a packaging mix are composed of a lentiviral vector plasmid that comprises at least LTRs (5' LTR and 3' LTR), a packaging signal (Ψ), and a target transgene and a plurality of plasmids that comprise gag, pol, rev, and env independently of each other.
[7] The method for producing a lentiviral vector according to [6], wherein the plurality of plasmids constituting a packaging mix are composed of 3 plasmids: (1) a lentiviral vector plasmid that comprises at least LTRs (5' LTR and 3' LTR), a packaging signal (Ψ), and a target transgene; (2) a packaging plasmid that comprises gag and pol necessary for packaging and may comprise rev and tat control genes; and (3) an envelope plasmid that comprises a gene encoding VSV-G.
[8] The method for producing a lentiviral vector according to [6], wherein the plurality of plasmids constituting a packaging mix are composed of 4 plasmids: (A) a lentiviral vector plasmid that comprises at least LTRs (5' LTR and 3' LTR), a packaging signal (Ψ), and a target transgene; (B) a packaging plasmid that comprises gag and pol necessary for packaging; (C) an envelope plasmid that comprises a gene encoding VSV-G; and (D) a rev expression plasmid that comprises rev.
[9] The method for producing a lentiviral vector according to [6], wherein the plurality of plasmids constituting a packaging mix are composed of 3 plasmids: (A) a lentiviral vector plasmid that comprises at least LTRs (5' LTR and 3' LTR), a packaging signal (Ψ), and a target transgene; (B) a packaging plasmid that comprises gag and pol necessary for packaging; and (C) a plasmid that comprises an envelope expression unit comprising a gene encoding VSV-G and a rev expression unit comprising rev.
[10] The method for producing a lentiviral vector according to [8] or [9], wherein 5' LTR and 3' LTR in the lentiviral vector plasmid are modified.
[11] The method for producing a lentiviral vector according to any of [3] to [10], wherein Tax is not incorporated into the lentiviral vector.
[12] A kit for producing a lentiviral vector in 293T cells comprising: a packaging mix comprising a plurality of plasmids that comprise genes encoding proteins essential for lentiviral particle formation and a plasmid that comprises a gene transcribed into RNA to be incorporated into lentiviral particles containing a transgene to be expressed in target cells; and an expression vector comprising a gene encoding a factor activating a promoter.
[13] The kit for producing a lentiviral vector according to [12], wherein the factor activating a promoter is a factor activating a CMV promoter.
[14] The kit according to [12] or [13], wherein the factor activating a promoter is one or more factors selected from the group consisting of HTLV-1 Tax, HIV-1 Tat, NF-κB RelA, AP-1, and CREB/ATF.
[15] The kit for producing a lentiviral vector in 293T cells according to any of [12] to [14], which comprises: a packaging mix comprising a plurality of plasmids that comprise genes encoding proteins essential for lentiviral particle formation and a plasmid that comprises a gene transcribed into RNA to be incorporated into lentiviral particles containing a transgene to be expressed in target cells; and an expression vector comprising a gene encoding HTLV-1 Tax or NF-κB RelA.
[16] The kit for producing a lentiviral vector in 293T cells according to any of [12] to [14], which comprises: a packaging mix comprising a plurality of plasmids that comprise genes encoding proteins essential for lentiviral particle formation and a plasmid that comprises a gene transcribed into RNA to be incorporated into lentiviral particles containing a transgene to be expressed in target cells; and an expression vector comprising genes encoding 2 types of factors selected from the group consisting of HTLV-1 Tax, HIV-1 Tat, and NF-κB RelA or 3 types of factors HTLV-1 Tax, HIV-1 Tat, and NF-κB RelA.
[17] The kit for producing a lentiviral vector according to [16], wherein the genes encoding 2 types of factors selected from the group consisting of HTLV-1 Tax, HIV-1 Tat, and NF-κB RelA and those encoding 3 types of factors HTLV-1 Tax, HIV-1 Tat, and NF-κB RelA are located on independent plasmid vectors for expression.
[18] The kit for producing a lentiviral vector according to any of [15] to [17], wherein the plurality of plasmids constituting a packaging mix are composed of a lentiviral vector plasmid that comprises at least LTRs (5' LTR and 3' LTR), a packaging signal (Ψ), and a target transgene and a plurality of plasmids that comprise gag, pol, rev, and env independently of each other.
[19] The kit for producing a lentiviral vector according to [18], wherein the plurality of plasmids constituting a packaging mix are composed of 3 plasmids: (1) a lentiviral vector plasmid that comprises at least LTRs (5' LTR and 3' LTR), a packaging signal (Ψ), and a target transgene; (2) a packaging plasmid that comprises gag and pol necessary for packaging and may comprise rev and tat regulatory genes; and (3) an envelope plasmid that comprises a gene encoding VSV-G.
[20] The kit for producing a lentiviral vector according to [18], wherein the plurality of plasmids constituting a packaging mix are composed of 4 plasmids: (A) a lentiviral vector plasmid that comprises at least LTRs (5' LTR and 3' LTR), a packaging signal (Ψ), and a target transgene; (B) a packaging plasmid that comprises gag and pol necessary for packaging; (C) an envelope plasmid that comprises a gene encoding VSV-G; and (D) a rev expression plasmid that comprises rev.
[21] The kit for producing a lentiviral vector according to [18], wherein the plurality of plasmids constituting a packaging mix are composed of 3 plasmids: (A) a lentiviral vector plasmid that comprises at least LTRs (5' LTR and 3' LTR), a packaging signal (Ψ), and a target transgene; (B) a packaging plasmid that comprises gag and pol necessary for packaging; and (C) a plasmid that comprises an envelope expression unit comprising a gene encoding VSV-G and a rev expression unit comprising rev.
[22] The kit for producing a lentiviral vector according to [20] or [21], wherein 5' LTR and 3' LTR in the lentiviral vector plasmid are modified.
[23] The 293T cell comprising the plasmid and the vector included in the kit according to any of [15] to [22].

This description includes part or all of the content as disclosed in the description and/or drawings of Japanese Patent Application No. 2018-176230, which is a priority document of the present application.

### [Effects of the Invention]

When a plurality of plasmids that are necessary for lentivirus packaging are cotransfected with 293T cells, either HTLV-1 Tax or NF-κB RelA is coexpressed, HTLV-1 Tax in combination with NF-κB RelA, HTLV-1 Tax in combination with HIV-1 Tat, or NF-κB RelA in combination with HIV-1 Tat are coexpressed, or 3 types of factors HTLV-1 Tax, NF-κB RelA, and HIV-1 Tat are coexpressed. Thus, lentiviral vector production can be increased. According to the method of the present invention, lentiviral vector production can be improved, and lentiviral vector production can be easily and safely performed. As a result, the cost required for performing the procedure including cell culture and transfection can be reduced. In addition, the method of the present invention can be performed in combination with current techniques of concentration and purification, so that such technique can be employed for a wide variety of *in vitro* and *in vivo* applications.

According to the method of the present invention, in addition, the Tax protein is not incorporated into lentiviral vector particles. Thus, safety of the products can be assured.

### [Brief Description of the Drawings]

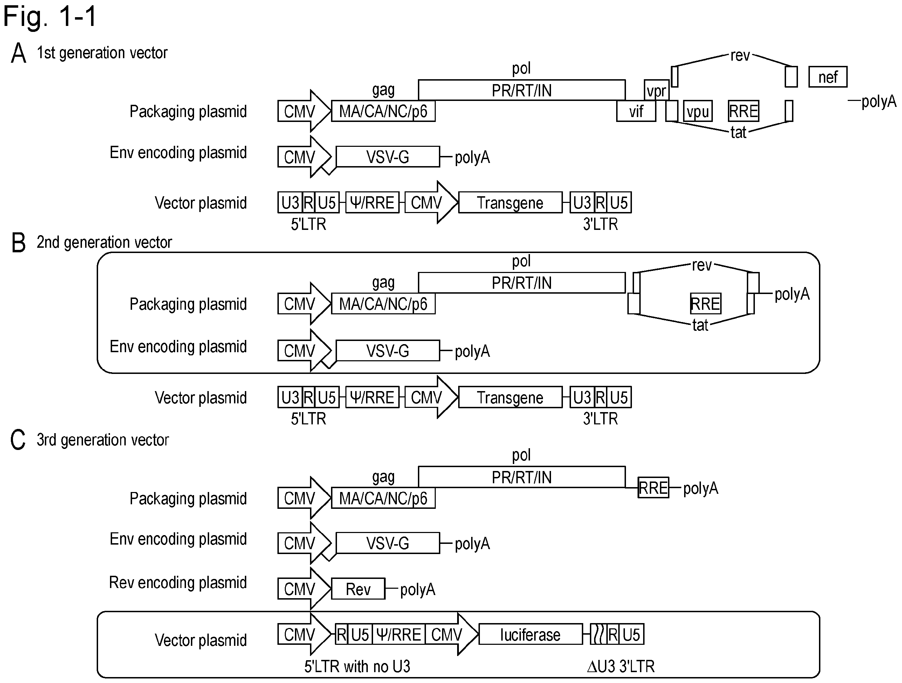

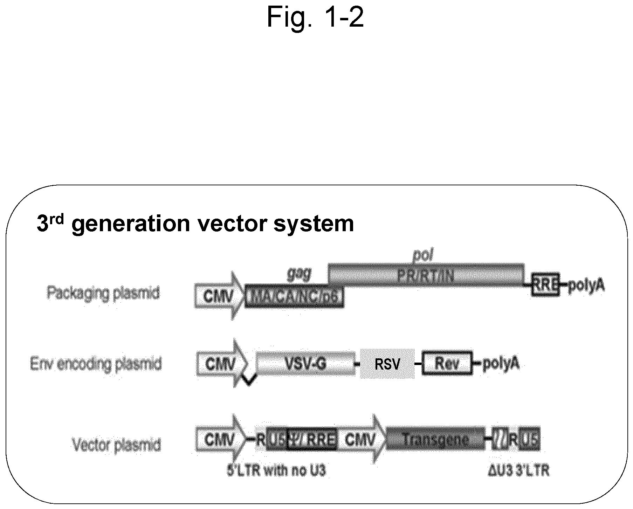

Fig. 1-1 shows structures of representative first-generation, second-generation, and third-generation lentiviral vector systems.
Fig. 1-2 shows a structure of a third-generation lentiviral vector system in which VSV-G and rev are included in a plasmid.
Fig. 2 demonstrates that Tax strongly activates a CMV promoter in the HEK293T cell.
Fig. 3 demonstrates increased lentiviral vector production achieved by coexpression of Tax in the lentiviral vector producer cell.
Fig. 4 shows Tax expression and Gag expression levels in the lentiviral vector producer cell.
Fig. 5 demonstrates increased virus release from the producer cell achieved by Tax.
Fig. 6 demonstrates that Tax is less likely to be incorporated into lentiviral vector particles.
Fig. 7-1 demonstrates effects of increasing lentiviral vector production when either HTLV-1 Tax (A), HIV-1 Tat (B), or NF-κB RelA (C) is coexpressed.
Fig. 7-2 demonstrates effects of increasing lentiviral vector production when HTLV-1 Tax in combination with HIV-1 Tat (A), HTLV-1 Tax in combination with NF-κB RelA (B), or NF-κB RelA in combination with HIV-1 Tat (C) or 3 types of factors HTLV-1 Tax, NF-κB RelA, and HIV-1 Tat (D) are coexpressed.
Fig. 8 shows a structure of the pSV plasmid.
Fig. 9 shows a structure of the transfer plasmid used in Example 2.
Fig. 10 demonstrates the results of increasing lentiviral vector production when either HIV-1 Tat or NF-κB RelA or both thereof are coexpressed.
Fig. 11 shows a structure of the transfer plasmid used in Example 3.
Fig. 12 shows a summary of a method of titration when Venus is used as a reporter gene.
Fig. 13 shows the results of titration when Venus is used as a reporter gene (an absolute titer).
Fig. 14 shows the results of titration when Venus is used as a reporter gene (an extent of titer increase).

### [Embodiments of the Invention]

Hereafter, the present invention is described in detail.

The present invention concerns a method for lentiviral vector production and a method for increasing the amount of lentiviral vector production.

In the method of the present invention, the 293T (HEK293T) cell is used as a packaging cell capable of lentiviral vector production, and a factor that acts on and activates a promoter is subjected to coexpression when producing lentivirus in the 293T cell. While a promoter is not limited, an example is a CMV promoter. Examples of a factor activating a promoter include Tax, NF-κB RelA, HIV-1 Tat (hereafter, referred to as "HIV-1 Tat" or "Tat"), AP-1, and CREB/ATF, while HIV-1 Tat (hereafter, referred to as "HIV-1 Tat" or "Tat") promotes elongation of transcribed RNA, and one of such factors may be subjected to coexpression, or a 2, 3, 4, or 5 of such factors may be subjected to coexpression in combination. In the present invention, at least one factor selected from among Tax, NF-κB RelA, and Tat is preferably subjected to coexpression as the factor activating a promoter or promoting transcriptional elongation.

In the method of the present invention, the 293T (HEK293T) cell is used as a packaging cell capable of lentiviral vector production, and a transcription factor, such as Tax or NF-κB RelA, Tat, or any thereof in combination are subjected to coexpression when producing the lentivirus in the 293T cell.

The lentiviral vector was developed based on human immunodeficiency virus type 1 (HIV-1).

In order to produce the lentiviral vector, an HIV-1 provirus genome may be divided and incorporated into a plurality of plasmids, and the plasmids may then be cotransfected (simultaneously transfected) into the cells. In this case, the entire virus genome is not necessarily divided and incorporated into a plurality of plasmids, and a part of the virus genome from which genes such as accessory genes have been removed may be incorporated therein. Specifically, a plurality of plasmids that comprise genes encoding proteins essential for lentiviral particle formation and a plasmid that comprises a gene transcribed into RNA to be incorporated into lentiviral particles and a target transgene (i.e., the lentiviral vector plasmid) may be cotransfected into the packaging cell. Examples of proteins essential for lentiviral particle formation include proteins such as Gag, Pol, Tat, and Rev essential for packaging, and an envelope (Env) protein such as VSV-G (the G glycoprotein of vesicular stomatitis virus). VSV-G has a wide range of hosts and is preferable from the viewpoint of high physical strength, although other envelope proteins can be used. The gag gene encodes the internal structural (an intercellular substance, capsid, or nucleocapsid) protein, the pol gene encodes RNA-dependent DNA polymerase (reverse transcriptase), protease, and integrase, and the env gene encodes a virus envelope glycoprotein. The lentiviral vector plasmid comprises a packaging signal (Ψ) and a target gene inserted therein.

Nucleic acids encoding proteins, such as Gag, Pol, Tat, and Rev, that are necessary for packaging and nucleic acids encoding an envelope (Env) protein, such as VSV-G, may be divided and incorporated into a plurality of plasmids, such as 3 to 5 plasmids, and preferably 3 or 4 plasmids, and such plurality of plasmids and the lentiviral vector plasmid may be cotransfected.

As a result of cotransfection, the recombinant virus RNA genome transcribed from the lentiviral vector plasmid is incorporated into the packaging protein Gag by the action of the packaging signal (Ψ), and the virus core is thus formed.

As described above, nucleic acids encoding proteins that are essential for virus formation are separately incorporated into different plasmids. This prevents wild-type HIV-1 from being generated via homologous recombination. The lentiviral vector that had infected the cell is not capable of autoreproduction in the cell. Accordingly, such vector does not repeat infection and is safe.

For example, a lentiviral vector plasmid, a plasmid comprising a nucleic acid encoding a protein, such as Gag, Pol, Tat, or Rev, and a plasmid comprising a nucleic acid encoding an envelope (Env) protein, such as VSV-G, can be used. Regulatory genes, such as rev and tat, may be separately incorporated into other plasmids.

Specifically, 3 plasmids: (1) a lentiviral vector plasmid that comprises at least LTRs (5' LTR and 3' LTR), a packaging signal (Ψ), and a target transgene; (2) a packaging plasmid that comprises gag and pol necessary for packaging and may comprise rev and tat regulatory genes; and (3) an envelope (Env) expression plasmid (envelope plasmid) comprising the env gene encoding an envelope such as VSV-G, can be used. Also, 4 plasmids: (A) a lentiviral vector plasmid that comprises at least LTRs (5' LTR and 3' LTR), a packaging signal (Ψ), and a target transgene; (B) a packaging plasmid that comprises gag and pol necessary for packaging and may comprise rev and tat control genes; (C) an envelope (env) expression plasmid (envelope plasmid) comprising the env gene encoding an envelope such as VSV-G; and (D) a rev expression plasmid that comprises rev, may be used. Alternatively, 3 plasmids: (A) a lentiviral vector plasmid that comprises at least LTRs (5' LTR and 3' LTR), a packaging signal (Ψ), and a target transgene; (B) a packaging plasmid that comprises gag and pol necessary for packaging; and (C) a plasmid comprising and env expression unit comprising the env gene encoding an envelope (env) such as VSV-G and a rev expression unit comprising rev, may be used.

5' LTR and 3' LTR may be modified. For example, U3 in 5' LTR and 3' LTR may be deleted or mutated. In such a case, U3 in 5' LTR may be substituted with a promoter such as a CMV promoter.

The plurality of plasmids used for lentiviral vector production in a cell constitute a packaging mix used for lentivirus production.

Preferably, a poly A addition sequence (a polyadenylation sequence; poly A) is ligated to the 3' terminuses of the plasmids other than the lentivirus vector plasmid. The origin of the poly A addition sequence (the polyadenylation sequence; polyA) is not limited, and examples of the poly A addition sequences include a poly A addition sequence derived from the growth hormone gene, such as a poly A addition sequence derived from the bovine growth hormone gene, a poly A addition sequence derived from the human growth hormone gene, a poly A addition sequence derived from the SV40 virus, and poly A addition sequences derived from human and rabbit β globin gene. By incorporating the poly A addition sequence into an expression cassette, RNA stability and translation efficiency are improved.

In the lentiviral vector plasmid, the internal promoter and the target gene are incorporated into a space between HIV and LTR at the both ends comprising the packaging signals (Ψ).

In the lentiviral vector plasmid and the packaging plasmid, RRE (the rev response element) may be incorporated.

A promoter may be ligated to the 5' terminus of each plasmid. As described above, in addition, the lentiviral vector plasmid comprises a promoter in a region upstream of the target gene. A promoter is not limited, and examples of promoters that can be used include CMV promoter, CMV-i promoter (hCMV + intron promoter), SV40 promoter, UbC promoter, EF1α promoter, RSV promoter, β actin promoter, CAG promoter, tissue-specific promoter, and tet expression controlling promoter. Among them, the CMV promoter is preferable.

It is preferable that at least one HIV accessory gene, such as vif, vpr, vpu, vpx, or nef, be removed or inactivated.

In addition, the lentiviral vector plasmid may comprise cPPT (the central polypurine tract) or WPRE (the woodchuck hepatitis virus post-transcriptional regulatory element). cPPT improves reverse transcription efficiency and WPRE improves RNA expression efficiency.

It is necessary that elements in plasmids are operably linked to each other. When such elements are operably linked to each other, each element exerts its function to increase the expression level of the gene to be expressed.

The lentiviral vector system for lentiviral vector production had been improved from the first-generation lentiviral vector system, the second-generation lentiviral vector system, to the third-generation lentiviral vector system, so as to prevent wild-type viruses being generated via homologous recombination.

The lentiviral vector system involving the use of the 3 plasmids (1) to (3) above is the second-generation lentiviral vector system, and the lentiviral vector system involving the use of the 4 plasmids (A) to (D) above is the third-generation lentiviral vector system.

In the third-generation lentiviral vector system, U3 in 5' LTR of the lentiviral vector plasmid may be substituted with the CMV promoter, and a mutation may be applied to U3 in 3' LTR, so as to neutralize the promoter activity thereof. Such lentiviral vector plasmid is referred to as the "self-inactivating (SIN)" plasmid. When U3 in 5' LTR is substituted with the CMV promoter, tat is reportedly not necessary, and tat can be removed from the packaging plasmid (MIYOSHI et al., J. Virol., 70, 8150, 1998; Dull et al., J. Virol., 72, 8463, 1998). The third-generation lentiviral vector is a tat-independent lentiviral vector system that does not comprise tat.

Fig. 1-1 shows structures of representative first-generation, second-generation, and third-generation lentiviral vector systems (modified from Biochem J., 2012, 443, 603-618). Fig. 1-2 shows a structure of a third-generation lentiviral vector system in which VSV-G and rev are included in a plasmid.

In the present invention, the second-generation lentiviral vector system or the third-generation lentiviral vector system is preferably used. The second-generation lentiviral vector system is advantageous in terms of the large amount of lentiviral vector production, and the third-generation lentiviral vector system is advantageous in terms of high safety.

When plasmids constituting the lentiviral vector system are cotransfected in 293T cells according to the method of the present invention, transcriptional activators, such as Tax and NF-κB RelA, Tat, or the like, are coexpressed in the cells.

Tax is a transcription activator of the human T cell leukemia virus type 1 (HTLV-1) virus gene: it activates transcription factors, such as AP-1, NF-κB, and CREB/ATF; the activated transcription factors bind to binding sequences in the promoters, such as the CMV promoters, to act on the promoters, promote the expression of the genes constituting the lentiviral vector system cotransfected into the 293T cells, and thus Tax increases the amount of lentiviral vector produced.

Tax may be coexpressed by cotransfecting 293T cell with an expression vector comprising a nucleic acid encoding the Tax protein, in addition to the plasmids constituting the lentiviral vector system. A virus vector other than the lentiviral vector, such as an adenovirus, adeno-associated virus, or gamma retrovirus vector, can be used as an expression vector. A promoter in the Tax expression vector is not limited, and CMV promoter, CMV-i promoter (hCMV + intron promoter), SV40 promoter, EF1α promoter, RSV promoter, β actin promoter, CAG promoter, tissue-specific promoter, or other promoters can be used. Expression may be transient or constitutive. Constitutive expression may be achieved by, for example, incorporating a nucleic acid encoding the Tax protein into the genome of the 293T cell.

HTLV-1 Tax is a potent virus trans-acting protein. If the trans-acting functions are sufficient, accordingly, incorporation thereof into the lentiviral vector particles may exert undesirable effects in the target cell. In the method of the present invention, incorporation of Tax into lentivirus particles is not observed.

In addition, a transcription factor that acts on a promoter such as the CMV promoter in the plasmids constituting the lentiviral vector system may be subjected to coexpression.

A promoter such as a CMV promoter comprises AP-1, NF-κB, and CREB/ATF recognition sites. Thus, Tax can be used for lentiviral vector production because Tax activates all such transcription factors. When a small amount of Tax is subjected to coexpression, Gag expression and lentiviral vector particle release are increased to a significant extent in producer cells, and transduction efficiency is improved by over 10 times. Examples of candidate transcription factors to be activated include AP-1, NF-κB, and CREB/ATF. As described above, Tax activates transcription factors, such as AP-1, NF-κB, and CREB/ATF, and the activated transcription factors act on a promoter such as the CMV promoter. Such transcription factors themselves may be coexpressed. When such transcription factors are coexpressed, the amount of transcription factors in the 293T cells increases, the transcription factors are more likely to be activated by Tax, expression of plasmids constituting the lentiviral vector system cotransfected in the 293T cells is accelerated as a consequence, and the increased lentiviral vector production can be expected. In such a case, NF-κB may express NF-κB RelA comprising a transcription activation domain.

Alternatively, Tat may be subjected to coexpression together with Tax or with Tax and transcription factors. As a result of coexpression of Tax and Tat, lentivirus production is additively increased. While the third-generation lentiviral vector system is reportedly a Tat-independent lentiviral vector system, the third-generation lentiviral vector system can increase lentiviral vector production via coexpression of Tax and Tat.

Expression of transcription factors and Tat can be performed in the same manner as with Tax expression. Specifically, an expression vector comprising nucleic acids encoding proteins is cotransfected into the 293T cell and coexpressed therein. In such a case, coexpression may be performed with the use of a plurality of vectors each comprising a different nucleic acid or a vector comprising 2 or 3 types of factors Tax, transcription factors, and Tat. The amount of each plasmid to be introduced may be adequately determined, nucleic acids encoding Tax, Tat, and NF-κB RelA may be transfected in an amount equivalent to or approximately 2 to 3 times in terms of the vector amount. When such plasmid is cotransfected into a 12-well plate, for example, the amount of a vector comprising a nucleic acid encoding Tax or Tat to be transfected may be approximately 0.1 µg/single well, and the amount of a vector comprising a nucleic acid encoding NF-κB RelA to be transfected may be approximately 0.1 to 0.2 µg/ single well.

When at least one of Tax, transcription factors, and Tat is coexpressed, the amount of lentivirus production is increased, and the amount of plasmids comprising genes encoding proteins essential for lentiviral particle formation can be thus reduced. With the use of plasmids in an amount 1/2 to 1/3 compared with the conventional technique (the method for lentiviral vector production, Hiroyuki Miyoshi, http://cfm.brc.riken.jp/wp-content/uploads/Subteam_for_Manipulation_of_Cell_Fate_J/Protocols_J_files/Lentiviral%20 vector%20prep.pdf), the amount of lentivirus production equivalent to or more than the conventional technique can be achieved for the following reasons. That is, the amount of plasmids used for expressing the lentiviral vector constituents may be reduced, so that the conditions of the lentiviral vector producer cell are improved, and the optimal effects can be attained by activating the CMV enhancer/promoter.

As described above, plasmids constituting the lentiviral vector system may be cotransfected into a packaging cell (the 293T cell), and Tax or Tax with Tat, and/or transcription factors may be coexpressed. Specifically, Tax by alone, RelA alone, Tax in combination with Tat, Tax in combination with RelA, Tat in combination with RelA, or Tax, Tat, and RelA in combination may be coexpressed.

The 293T (HEK293T) cell is derived from the 293 cell (HEK293 cell), which is a cell line derived from the human embryonic kidney, and it is a cell line expressing the SV40 Large T antigen. The 293T cell is commercially available and it can be obtained from, for example, ATCC (American Type Culture Collection). In the present invention, a cell derived from the 293T cell can be used, and an example thereof is the Lenti-X⁽™⁾ 293T cell (TaKaRa Bio Inc.). In the present invention, such 293T cell-derived cell is also referred to as the "293T cell."

The 293T cells can be transfected with plasmids in accordance with a conventional technique. Examples of conventional techniques include a method involving the use of a transduction reagent, the calcium phosphate method, lipofection, the DEAE dextran method, electroporation, and microinjection. Examples of transduction reagents include polyethyleneimine (PEI, linear or branched) and commercially available transduction reagents. Examples of commercially available transduction reagents include Lipofectamine®, Lipofectamine plus®, jet PEI®, TransIT®, Xfect^{(TM)}, Transfectin-Lipid®, Oligofectamine®, siLentFect®, DMRIE-C®, Effectene®, and FuGENE®. Any of linear polyethyleneimine (PEI) and branched PEI containing primary, secondary, and tertiary amines can be used. The molecular amount of PEI is not limited. In addition, PEI subjected to chemical modification such as diacylation can be used.

After cotransfection in 293T cells, culture is conducted for several days: the culture supernatant is recovered, and lentivirus particles are then recovered from the culture supernatant. The medium and the conditions for conventional animal cell culture are employed for 293T cell culture. A culture period for lentivirus production is, for example, 12 to 72 hours, and preferably 24 to 48 hours. In order to recover virus vectors having sufficient titers, it is preferable that the culture supernatant titer be measured. The "sufficient titer" used herein is 1 × 10⁵ IFU/ml or higher, and preferably 5 × 10⁵ IFU/ml or higher. The titer can be measured via real-time RT-PCR, p24 ELISA, flow cytometry, or other means. The amount of the resulting lentiviral vectors can be determined on the basis of transduction efficiency into cells.

According to the method of the present invention, the amount of lentivirus production is increased by at least 3 fold, preferably 5 fold, more preferably 8 fold, more preferably 12 fold, more preferably 14 fold, more preferably 16 fold, more preferably 18 fold, more preferably 20 fold, more preferably 22 fold, and more preferably 24 fold, relative to the transduction efficiency achieved by control transfection as determined by, for example, luciferase reporter assays. Higher effects of increased production are attained upon coexpression of 2 or 3 of Tax, Tat, and transcription factors. Upon coexpression of Tax and Tat, production is increased by at least 5 fold. Upon coexpression of Tax and RelA, production is increased by at least 12 fold. Upon coexpression of Tat and RelA, production is increased by at least 16 fold. Upon coexpression of Tax, Tat, and RelA, production is increased by at least 22 fold.

The recovered lentiviral vector can be concentrated and purified via ultracentrifugation or other means.

The resulting lentiviral vector may be cryopreserved.

The lentiviral vector thus obtained can be used for gene introduction into mammalian cells that are either growing cells or growth-arrested cells. The introduced genes are incorporated stably in the host cell genome, and gene expression can be maintained for a long period of time. Accordingly, such lentiviral vector can be preferably used as a vector for gene therapy.

The present invention encompasses a reagent or kit for increasing lentiviral vector production using 293T cells.

The reagent or kit comprises the packaging mix comprising a plurality of plasmids that comprise genes encoding proteins essential for lentiviral particle formation and a plasmid that comprises a gene transcribed into RNA to be incorporated into lentiviral particles and a target transgene (the lentiviral vector plasmid) and an expression vector expressing HTLV-1 Tax. The reagent or kit may further comprise an expression vector expressing a transcription factor selected from the group consisting of AP-1, NF-κB, and CREB/ATF and/or an expression vector expressing HIV-1 Tat.

### [Examples]

The present invention is described in greater detail with reference to the following examples, although the present invention is not limited to these examples.

### [Example 1] Increasing lentiviral vector production with HTLV-1 Tax

### Materials and method

### Cells

The HEK293T cells (Invitrogen Corp., Carlsbad, CA) were cultured in the Dulbecco's modified Eagle medium (NakaleiTesque) supplemented with 10% fetal bovine serum (FBS), 100 U/ml penicillin, and 100 µg/ml streptomycin. The MT-4 cells were maintained in the complete RPMI 1640 medium (NakaleiTesque) supplemented with 10% FBS, 100 U/ml penicillin, and 100 µg/ml streptomycin.

### Plasmids

pCSII-CMV-MCS-IRES2-Bsd, pCAG-HIVgp, and pCMV-VSV-G-RSV-Rev were provided by RIKEN BRC through the National BioResource Project (NBRP) directed by the Ministry of Education, Culture, Sports, Science and Technology. In order to generate pCSII-CMV-luc-IRES2-Bsd, pCSII-CMV-MCS-IRES2-Bsd and pCMV-luc were digested with *Xho*I and *Not*I*.* Subsequently, the *Xho*I*-Not*I fragment carrying the firefly luciferase gene derived from pCMV-luc was inserted into the *Xho*I*-Not*I site of pCSII-CMV-MCS-IRES2-Bsd. The obtained plasmid was designated as pCSII-CMV-luc-IRES2-Bsd. pHCMV-VSV-G was provided by I. S. Y. Chen. pCMVdeltaR8.2, pCMV-Neo-Bam-Tax, and pCMV-Neo-Bam was prepared in accordance with the method described in Yamaoka, S. et al., Cell 93, 1231-1240, 1998; Naldini, L. et al., Proceedings of the National Academy of Sciences of the United States of America 93, 11382-11388, 1996; Hironaka, N. et al., Neoplasia 6, 266-278, doi:10.1593/neo.03388, 2004; and Baker, S. J. et al., Science 249, 912-915, 1990.

pH2RneoTax, pSV2Tat, and pRC/CMVRelA were prepared in accordance with the method described in Yamaoka et al., EMBO J. 15, 873-87, 1996; Subramani S et al., Mol. Cell. Biol., 1, 854, 1981; and Schmitz and Baeuerle, EMBO J., 10, 3805-3817, 1991.

### Western blotting

A lysis buffer (25 mM Tris, pH 7.8, 8 mM MgCl₂, 1 mM DTT, 1% Triton-X100, and 15% glycerol) was used to prepare a cell lysate. Protein concentration was determined by Bradford assays. Proteins were separated via SDS-PAGE, transferred to a polyvinylidene fluoride (PVDF) membrane, and then allowed to react with the mouse monoclonal antibody against Tax (MI73) (Mori, K. et al., The Journal of general virology 68 (Pt 2), 499-506, doi: 10.1099/0022-1317-68-2-499, 1987), the mouse monoclonal antibody against HIV-1 p24 (39/5.4A, Abcam, Inc.), the rabbit polyclonal antibody against Cyclophilin A (BML-SA296, Enzo Life Sciences, Inc.), or the mouse monoclonal antibody against α-tubulin (DM1A, Sigma-Aldrich Co.). Thereafter, the membrane was subj ected to incubation together with horseradish peroxidase-binding rabbit anti-mouse IgG (A206PS, American Qualex International Inc.) or goat anti-rat IgG (sc-2006, Santa Cruz Biotechnology), and the protein was visualized using Western Lightning Plus-ECL (PerkinElmer).

### Preparation of viral solution

In order to produce the lentiviral vector in the presence of Tax, 1.4 µg of pCSII-CMV-luc-IRES2-Bsd, 0.9 µg of pCMVdeltaR8.2, and 0.4 µg of pHCMV-VSV-G were cotransfected with pCMV-Neo-Bam-Tax and/or pCMV-Neo-Bam per 6-well plate using polyethyleneimine (PEI). The viruses in the supernatant were harvested 48 hours after transfection. The amount of HIV-1 capsids (CA) in the supernatant was quantified via HIV-1 CA (p24) ELISA (enzyme-linked immunosorbent assay) (ZeptMetrix Corporation).

Either Tax, Tat, or RelA was subjected to coexpression by itself, in combinations of two, or in combinations of all three thereof. Effects of the sole coexpression of Tax, Tat, or RelA, the dual coexpression of any of Tax, Tat, and RelA, and the triple coexpression of all of Tax, Tat, and RelA on the amount of the third-generation lentiviral vector production were examined by cotransfecting 0.14 µg of pCAG-HIVgp, 0.14 µg of pCMV-VSV-G-RSV-Rev, or 0.24 µg of pCSII-CMV-luc-IRES2-Bsd together with 0.05 µg of expression vectors therefor or control empty vectors (EV1, EV2, and EV3) per 24-well plate using PEI.

### Luciferase reporter assay

The MT4 cells (approximately 2 × 10⁵ cells) in the 24-well plate infected with lentiviral vector were recovered 24 hours after infection, lysed with a lysis buffer (25 mM tris, pH 7.8, 8 mM MgCl₂, 1 mM DTT, 1% Triton-X100, and 15% glycerol), and then subjected to luciferase activity assays. The firefly luciferase activity was assayed using the GloMax-Multi Detection system (Promega Corp.) in accordance with the protocols provided by the manufacturer.

Luciferase activity was standardized relative to the protein concentration determined by the Bradford assays.

### Purification of lentiviral vector

A 20% sucrose solution (2 ml) was introduced into the bottom of a ultracentrifuge tube (model: SW55), and 2 ml of the lentiviral vector-containing culture supernatant was superposed thereon. Subsequently, the sample was centrifuged at 4°C and 35,000 rpm for 60 minutes. A fraction that can be pelletized was resuspended in PBS(-) and was then subjected to Western blotting.

### Results

HTLV-1 Tax increases lentiviral vector production to a significant extent.

Lentiviral vector production using HEK293T cells was performed by cotransfection of firefly luciferase (pCSII-CMV-luc-IRES2-Bsd) and a lentivirus transfer vector capable of expressing the packaging plasmid (pCMV deltaR8.2) and a plasmid expressing the glycoprotein of vesicular stomatitis virus (pHCMV-VSV-G) together with CV (a control vector; an empty vector) into HEK293T cells.

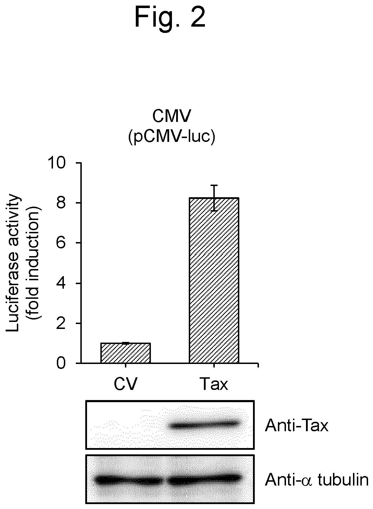

Further, it was deduced that a protein that would simultaneously activate AP-1, NF-κB, and CREB/ATF would efficiently increase lentivirus production. Such candidate protein is HTLV-1 Tax and is known to activate several cell transcription factors including AP-1, NF-κB, and CREB/ATF19 (Currer et al., Frontiers in Microbiology, 3, article 406, 2012). The research that had been conducted in the past reports indicated that Tax activated a CMV promoter in HEK293 cells but did not activate a CMV promoter in HEK293T cells (Lwa, T.R. et al., Biotechnology progress 27, 751-756, doi:10.1002/btpr.571, 2011). However, the present inventors discovered that Tax would strongly activate the CMV promoter in HEK293T cells. Specifically, Tax was cotransfected with 0.1 µg of pCMV-luc as a reporter vector and 0.2 µg of pCMV-Neo-Bam-Tax (Tax) as an effector vector or pCMV-Neo-Bam as a control vector (CV) into approximately 2 × 10⁵ HEK293T cells. The cell lysate (30 µg) was subjected to Western blotting using the anti-HTLV-1 Tax or anti-α-tubulin antibody (the lower panel in Fig. 2). The upper panel in Fig. 2 demonstrates the results of luciferase assays as the fold change compared with CV. A bar demonstrates a standard error calculated based on 3 independent experiments.

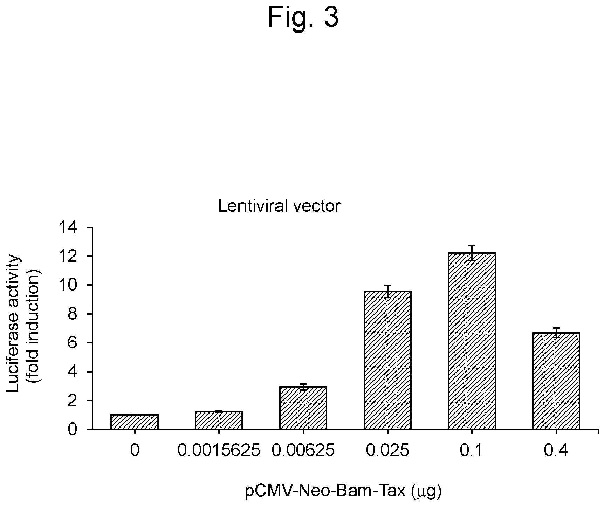

To approximately 1.5 × 10⁶ HEK293T cells, 0.4 µg of pHCMV-VSV-G, 0.9 µg of pCMVΔR8.2, and 1.4 µg of pCSII-CMV-luc-IRES2-Bsd were cotransfected with CV or successively increased pCMV-Neo-Bam-Tax. The total amount of effector plasmids was adjusted to 0.4 µg. The lentiviral vector-containing culture supernatant was sampled 48 hours after transfection. Transduction efficiency into the MT-4 cells exposed to the culture supernatant was evaluated 24 hours after exposure. Luciferase activity was corrected using protein concentration, and the results were expressed as the fold change compared with the control (0 µg of pCMV-Neo-Bam-Tax). The mean and the standard error calculated based on the 3 independent experiments are shown. Hereafter, the results of reporter assays were corrected using protein concentration. As demonstrated by the luciferase activity in the infected cells shown in Fig. 3, Tax coexpression in the lentiviral vector producer cell increased lentiviral vector production by up to 12 fold.

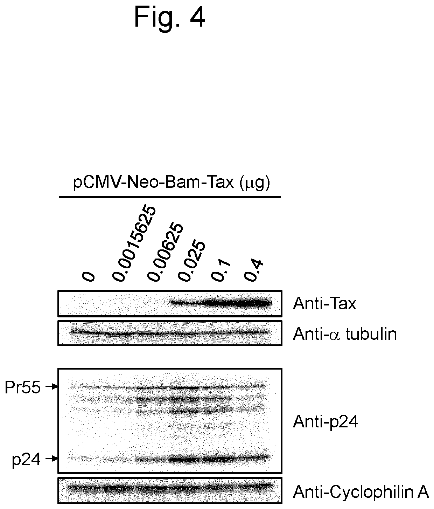

The transfected cells shown in Fig. 3 were recovered 48 hours after transfection. The same set of the cell lysate (30 µg) was subjected to Western blotting using the anti-HTLV-1 Tax and anti-α-tubulin antibodies or the anti-HIV-1p24 and anti-Cyclophilin A antibodies. As shown in Fig. 4, dose-dependent increase in Tax expression was observed via Western blotting in the producer cells. The Gag expression level in the producer cells was highly correlated with the results of lentiviral vector transduction.

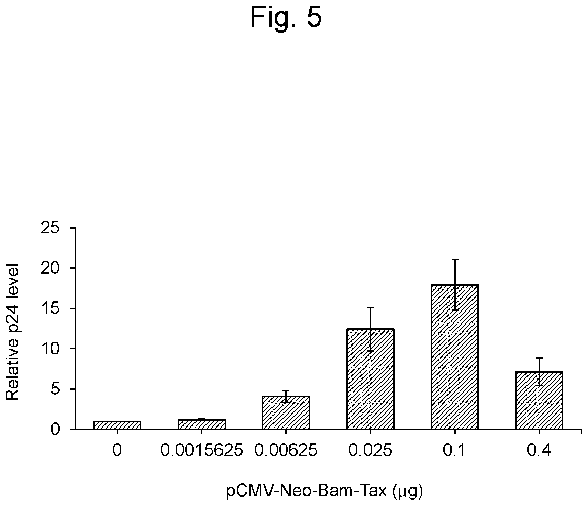

In order to evaluate whether or not Tax would increase virus release from the producer cells, HIV-1 Gag concentration was measured via specific ELISA. The amount of the Gag protein in the supernatant of the transfected cells was quantified via HIV-1 CA (p24) ELISA, and the results were expressed as the fold change compared with the control (0 µg of pCMV-Neo-Bam-Tax). The mean and the standard error calculated based on the 3 independent experiments are shown. As shown in Fig. 5, the amount of Gag in the supernatant also increased in the presence of Tax and it was highly correlated with transduction efficiency and the Gag expression level in the producer cells (Fig. 3 and Fig. 4).

In the end, whether or not Tax had been incorporated into lentiviral vector particles was examined from the viewpoint of safety and decontamination.

HTLV-1 Tax is a potent virus trans-acting protein. If the trans-acting functions are sufficient, accordingly, incorporation thereof into the lentiviral vector particles may exert undesirable effects in the target cell.

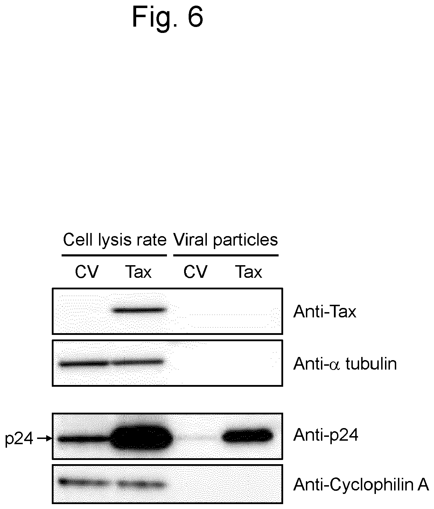

The viral particles in the culture supernatant were recovered via ultracentrifugation in the form of a pellet using a sucrose cushion. Specifically, lentiviral particles were recovered via ultracentrifugation using 20% sucrose. Subsequently, the cell lysate and the pelletized viral particles were subjected to Western blotting. The same set of the cell lysate (30 µg) was subjected to Western blotting using the anti-HTLV-1 Tax and anti-α-tubulin antibodies or the anti-HIV-1p24 and anti-Cyclophilin A antibodies. As shown in Fig. 6, no Tax protein was detected via Western blotting of the pellet obtained via ultracentrifugation. The results indicate that Tax is highly unlikely to be incorporated into lentiviral vector particles.

This example demonstrates that coexpression of transcription activators such as HTLV-1 Tax would increase the viral protein expression in the producer cells to enhance lentiviral vector production to a significant extent (Figs. 2 to 6). Tax increased the CMV promoter-driven reporter gene expression (Fig. 2) and further increased Gag expression in the producer cells. It was highly correlated with the results of lentiviral vector transduction (Figs. 3 and 4). In the experimental environment in this example, the CMV promoter drives a lentivirus transfer vector (pCSII-CMV-luc-IRES2-Bsd), a packaging plasmid (pCMVdeltaR8.2), and a plasmid expressing an envelope (pHCMV-VSV-G). Accordingly, the results demonstrated above strongly indicate that increased gene expression from such plasmids leads to efficient lentiviral vector production. Officially, however, Tax may activate expression of a certain type of host HIV-1-dependent factor and contribute to increased lentivirus expression. It should be noted that the level of lentiviral vector production was lowered from its peak upon cotransfection of 0.4 µg of the Tax expression vector (Fig. 3). The results demonstrate that an excess plasmid would adversely affect lentiviral vector production. Under such conditions, Gag expression actually decreased in the producer cells (Fig, 4).

### [Example 2] Increasing lentiviral vector production by sole expression of HTLV-1 Tax, Tat, or NF-κB RelA or coexpression of all thereof

Subsequently, effects of increasing lentiviral vector production achieved by coexpression of the third-generation lentiviral vector systems pCAG-HIVgp, pCMV-VSV-G-RSV-Rev, and pCSII-CMV-luc-IRES2-Bsd in the following combinations was examined using the luciferase activity in the MT4 cells as the indicators (Fig, 7).

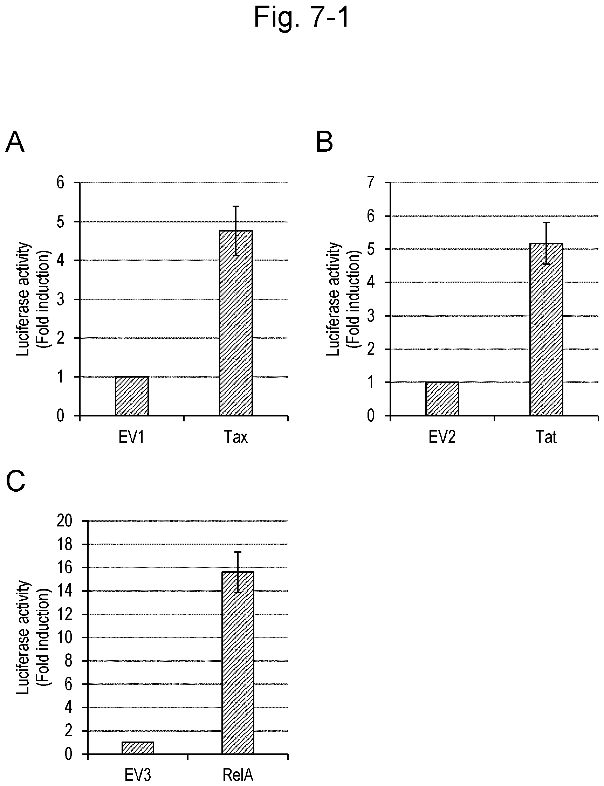

Fig. 7-1 shows effects of the sole coexpression of Tax (Fig. 7-1A), the sole coexpression of Tat (Fig. 7-1B), and the sole coexpression of RelA (Fig. 7-1C) on the amount of third-generation lentiviral vector production.

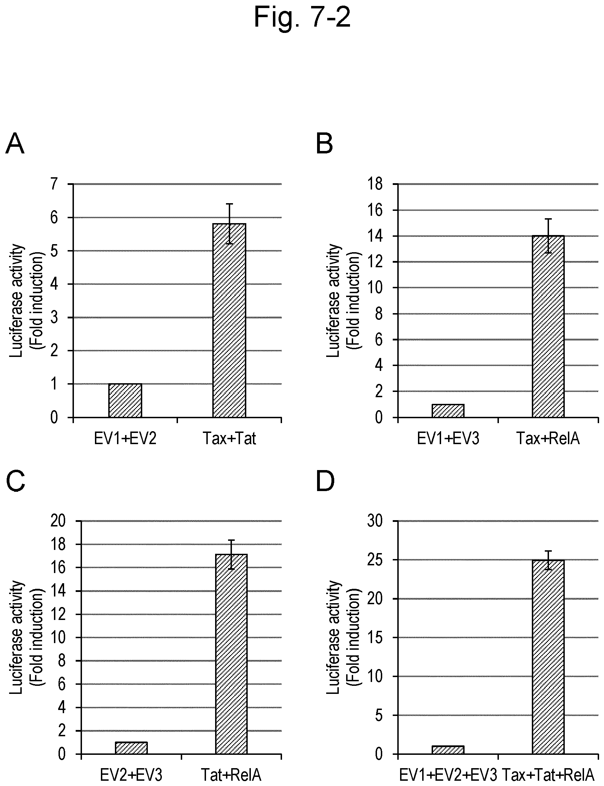

Fig. 7-2 shows effects of the dual coexpression of factors selected from among Tax, Tat, and RelA (Fig. 7-2A: Tax in combination with Tat; Fig. 7-2B: Tax in combination with RelA; and Fig. 7-2C: Tat in combination with RelA) on the amount of third-generation lentiviral vector production.

Fig. 7-2D shows effects of the triple coexpression of Tax, Tat, and RelA on the amount of third-generation lentiviral vector production.

In the case of the sole coexpression, as shown in Fig. 7-1, lentiviral vector production was increased by 4.8 fold with Tax, 5.1 fold with Tat, and 15.6 fold with RelA. In the case of the dual coexpression, as shown in Fig. 7-2, lentiviral vector production was increased by 5.8 fold with Tax in combination with Tat, 14.0 fold with Tax in combination with RelA, and 17.1 fold with Tat in combination with RelA. In the case of the triple coexpression, lentiviral vector production was increased by 24.9 fold with Tax, Tat, and RelA in combination. A "fold change" indicates a fold induction compared with the value attained with the use of empty vectors (EV1, EV2, and EV3).

### [Example 3] Increasing lentiviral vector production by sole expression of Tat or NF-κB RelA or coexpression thereof

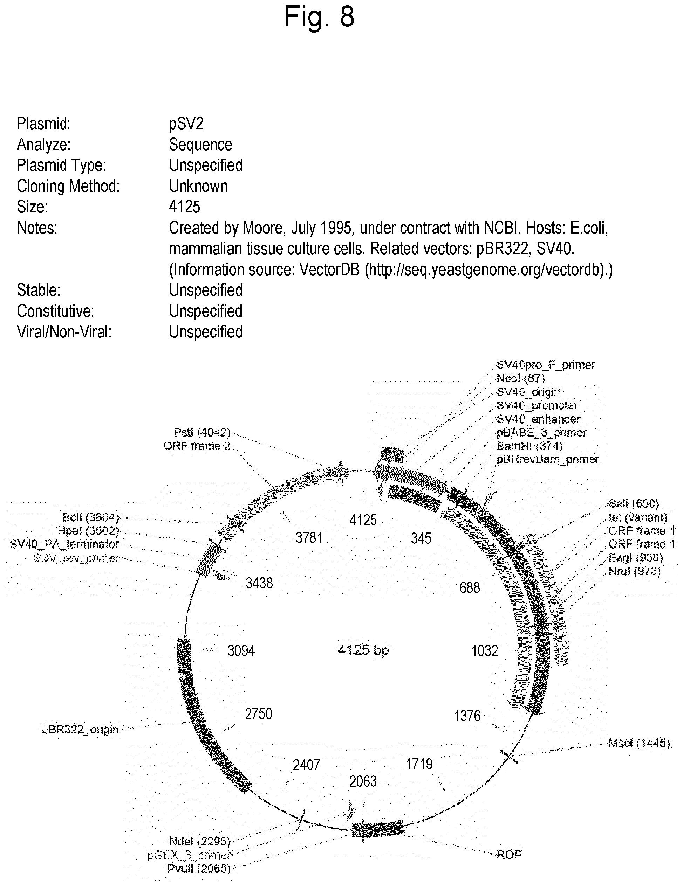

(1) Human embryonic kidney (HEK) 293T cells were inoculated and adhered to a collagen-coated 24-well plate at 3.0 × 10⁵ cells/well using 0.5 ml of DMEM supplemented with 10% fetal bovine serum.
(2) The plasmids shown below were mixed with 900 µl of Opti-MEM (Gibco) 2 hours later, 30 µl of 1 µg/µl polyethylenimine (PEI) was added thereto, the mixture was incubated for 30 minutes at room temperature, and the resultant was added dropwise to the cells.
   (i) 0.25 µg of pCAG-HIVgp
   (ii) 0.125 µg of pCMV-VSV-G-RSV-Rev
   (iii) 0.375 µg of pCSII-CMV-luc-IRES2-Bsd
   (iv) 0.05 µg of pSV2Tat or pSV2
   (v) 0.025 µg of pRC/CMVRelA or pRC/CMV (Invitrogen)
      The structure of the pSV2 plasmid is shown in Fig. 8.
(3) The culture solution was exchanged 24 hours later.
(4) The lentiviral vector-containing culture solution was recovered 48 hours later and filtered through a 0.22-µm filter (Millex). Immediately thereafter, 2 × 10⁵ MT4 cells (human T cells) were infected with 100 µl thereof.
(5) The MT4 cells were recovered via centrifugation 24 hours later and dissolved in 50 µl of a buffer containing 1% Triton X (25 mM tris, pH 7.8, 8 mM MgCl_{2,}1 mM DTT,1% Triton-X100, and 15% glycerol). Following centrifugation, 20 µl of the resultant was subjected to luciferase assays in the same manner as in Example 2, and assays were performed using the GloMax-Multi Detection system in accordance with the protocols provided by the manufacturer.

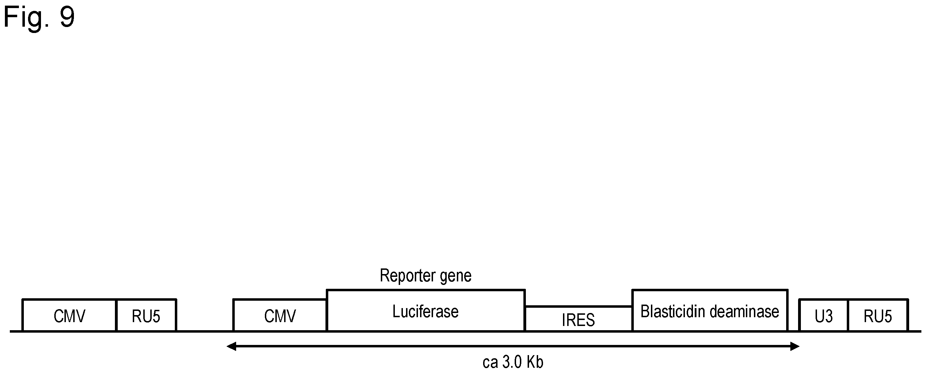

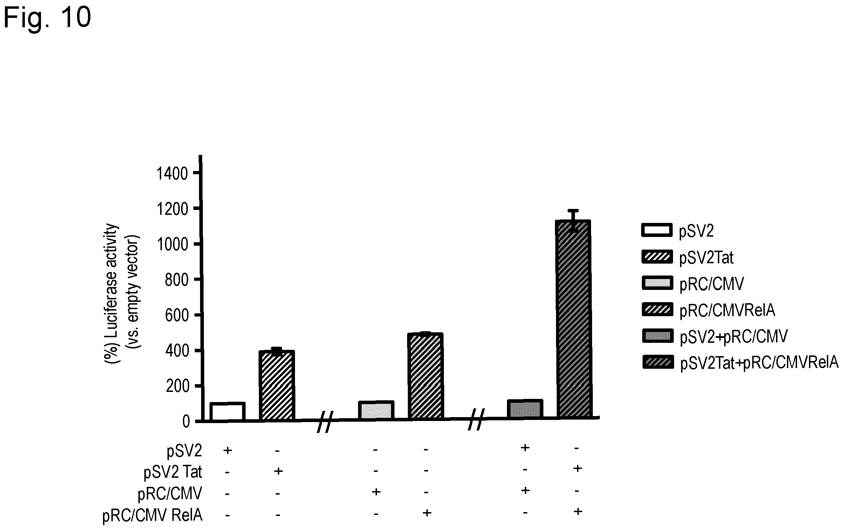

Fig. 9 shows a structure of the transfer plasmid (iii) (expressing the genome of the virus vector comprising the transgene). Fig. 10 shows the results of luciferase assays. As shown in Fig. 10, gene (luciferase) expression levels increased by approximately 4 fold upon sole expression of Tat, by approximately 5 fold upon sole expression of RelA, and by approximately 11 fold upon coexpression of Tat in combination with RelA in the target cells to be infected.

### [Example 4] Titers measured upon coexpression of Tat, RelA, and Tat in combination with RelA with the use of Venus as a reporter gene

(1) Human embryonic kidney (HEK) 293T cells were inoculated and adhered to a collagen-coated 24-well plate at 3.0 × 10⁵ cells/well using 0.5 ml of DMEM supplemented with 10% fetal bovine serum.
(2) The plasmids shown below were mixed with 900 µl of Opti-MEM 2 hours later, 30 µl of 1 µg/µl polyethylenimine (PEI) was added thereto, the mixture was incubated for 30 minutes at room temperature, and the resultant was added dropwise to the cells.
   (i) 0.25 µg of pCAG-HIVgp
   (ii) 0.125 µg of pCMV-VSV-G-RSV-Rev
   (iii) 0.375 µg of pCSII-CMV-HSVTK -IRES2-Venus
   (iv) 0.05 µg of pSV2Tat or pSV2
   (v) 0.025 µg of pRC/CMVhRelA or pRC/CMV
      pCSII-CMV-HSVTK -IRES2-Venus was prepared in the manner described below.
      The TK (thymidine kinase) DNA fragment derived from HSV-1 amplified via PCR with the use of pNL4-3tk (Hori et al., JVI 2013) as a template was inserted into a site between the *Nhe*I recognition site and the *Bam*HI recognition site of CSII-CMV-RfA-IRES2-Venus (from Dr. Hiroyuki Miyoshi, RIKEN).
      The primers shown below were used for PCR.
      5'-TK in CSII *Nhe*I: GCTCTAGAGCTAGCATGGCTTCGTACCCCTGCCATCAACAC(SEQ ID NO: 3)
      3'-TK in CSII *Bam*HI: CGCGGATCCTCAGTTAGCCTCCCCCATCTCC (SEQ ID NO: 4)
(3) The culture solution was exchanged 24 hours later.
(4) The lentiviral vector-containing culture solution was recovered 48 hours later and filtered through a 0.22-µm filter. Immediately thereafter, the culture supernatant was serially diluted two-fold from 1/8 to 1/64, and 1 × 10⁵ MT4 cells (human T cells) were infected with 10 µl thereof.
(5) The ratio of Venus fluorescence-positive cells was determined using FACS analyzer (FACSCalibur, BECTON DICKINSON) 48 hours later.

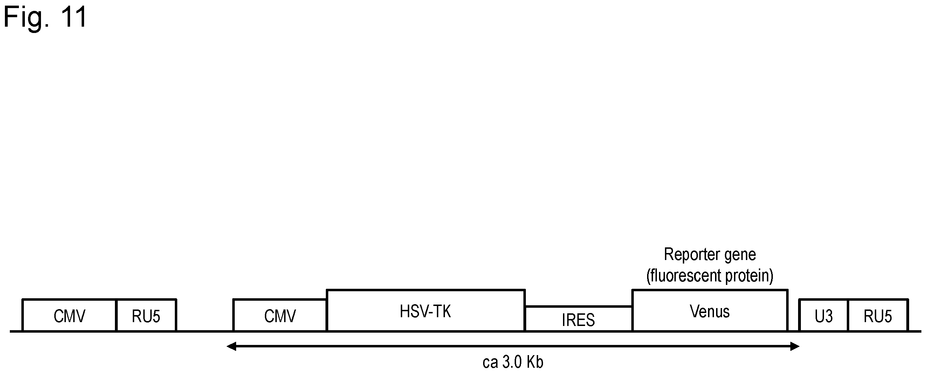

Fig. 11 shows a structure of the transfer plasmid (iii) (expressing the genome of the virus vector comprising the transgene).

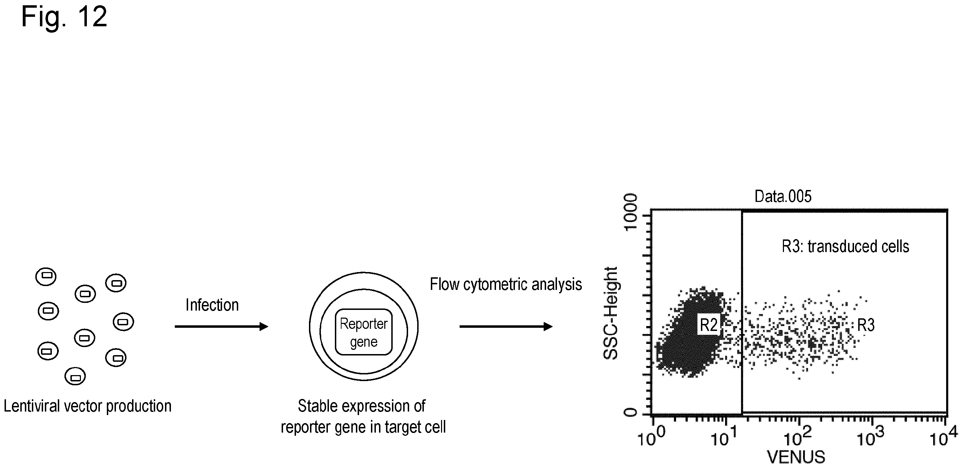

Fig. 12 shows a summary of the method of titration using Venus as a reporter gene.

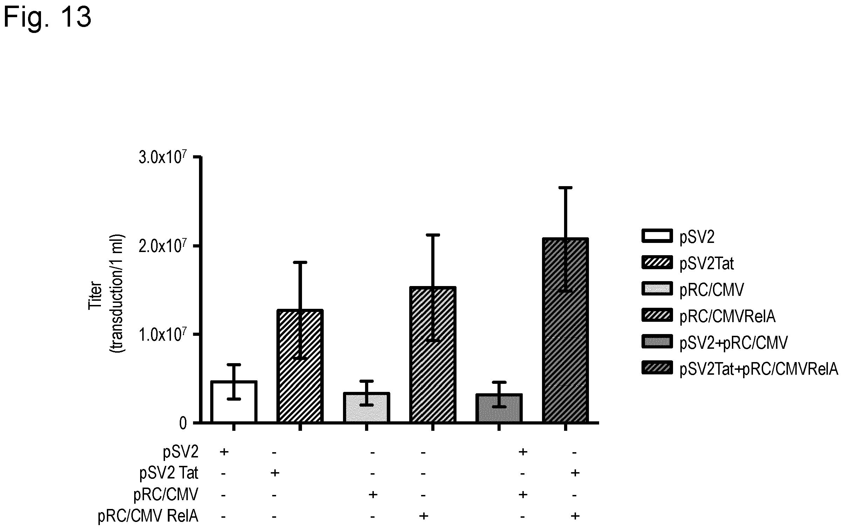

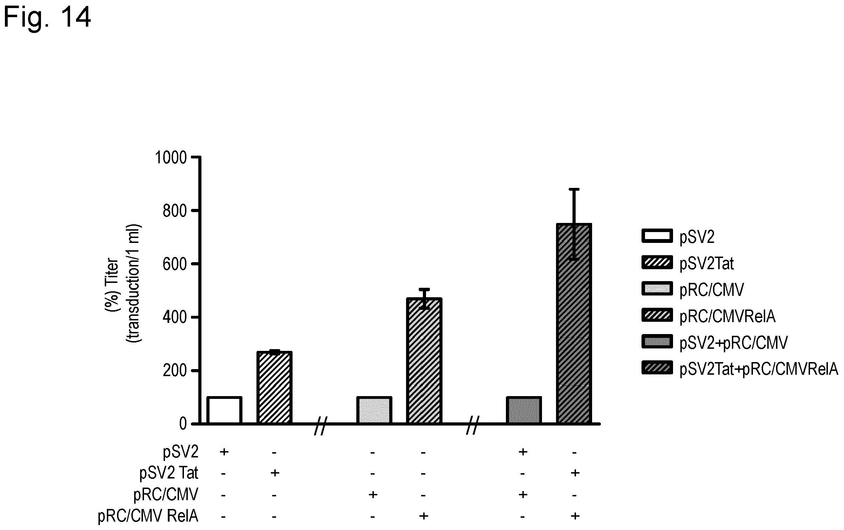

Fig. 13 and Fig. 14 show the results of titration. Fig. 13 shows the absolute titer (the titer of the lentiviral vector), and Fig. 14 shows an extent of titer increase compared with the titer measured with the use of the control vector (100%). As shown in Fig. 13 and Fig. 14, the titer increased by approximately 3 fold upon sole expression of Tat, by approximately 4 fold upon sole expression of RelA, and by approximately 6 fold upon coexpression of Tat in combination with RelA, and the titer as high as 2 × 10⁷ transduction unit (TU)/l was attained.

### [Industrial Applicability]

According to the method of the present invention, the lentiviral vector for gene introduction that can be used for gene therapy or other purposes can be produced with safety and high efficiency.

### [Sequence Listing Free Text]

SEQ ID NO: 1: Primer
SEQ ID NO: 2: Primer
SEQ ID NO: 3: Primer
SEQ ID NO: 4: Primer

All publications, patents, and patent applications cited herein are incorporated herein by reference in their entirety.

## Claims

1. A method for producing a lentiviral vector that enables substantial increase in lentiviral vector production, when cotransfecting 293T cells with a packaging mix comprising a plurality of plasmids that comprise genes encoding proteins essential for lentiviral particle formation and a plasmid that comprises a gene transcribed into RNA to be incorporated into lentiviral particles containing a transgene to be expressed in target cells, by simultaneously expressing factors which can activate promoters of transfected genes.

2. The method for producing a lentiviral vector according to Claim 1, wherein the factor activating a promoter is a factor activating a CMV promoter.

3. The method according to Claim 1 or 2, wherein the factor activating a promoter is one or more factors selected from the group consisting of HTLV-1 Tax, HIV-1 Tat, NF-κB RelA, AP-1, and CREB/ATF.

4. The method for producing a lentiviral vector according to any one of Claims 1 to 3, which comprises, when cotransfecting 293T cells with a packaging mix comprising a plurality of plasmids that comprise genes encoding proteins essential for lentiviral particle formation and a plasmid that comprises a gene transcribed into RNA to be incorporated into lentiviral particles containing a transgene to be expressed in target cells, by simultaneously expressing HTLV Tax or NF-κB RelA which can activate promoters of transfected genes in the 293T cells.

5. The method for producing a lentiviral vector according to any one of Claims 1 to 3, which comprises, when cotransfecting 293T cells with a packaging mix comprising a plurality of plasmids that comprise genes encoding proteins essential for lentiviral particle formation and a plasmid that comprises a gene transcribed into RNA to be incorporated into lentiviral particles containing a transgene to be expressed in target cells, by simultaneously expressing 2 types of factors selected from the group consisting of HTLV-1 Tax, HIV-1 Tat, and NF-κB RelA or 3 types of factors HTLV-1 Tax, HIV-1 Tat, and NF-κB RelA which can activate promoters of transfected genes in the 293T cells.

6. The method for producing a lentiviral vector according to Claim 4 or 5, wherein the plurality of plasmids constituting a packaging mix are composed of a lentiviral vector plasmid that comprises at least LTRs (5' LTR and 3' LTR), a packaging signal (Ψ), and a target transgene and a plurality of plasmids that comprise gag, pol, rev, and env independently of each other.

7. The method for producing a lentiviral vector according to Claim 6, wherein the plurality of plasmids constituting a packaging mix are composed of 3 plasmids: (1) a lentiviral vector plasmid that comprises at least LTRs (5' LTR and 3' LTR), a packaging signal (Ψ), and a target transgene; (2) a packaging plasmid that comprises gag and pol necessary for packaging and may comprise rev and tat control genes; and (3) an envelope plasmid that comprises a gene encoding VSV-G.

8. The method for producing a lentiviral vector according to Claim 6, wherein the plurality of plasmids constituting a packaging mix are composed of 4 plasmids: (A) a lentiviral vector plasmid that comprises at least LTRs (5' LTR and 3' LTR), a packaging signal (Ψ), and a target transgene; (B) a packaging plasmid that comprises gag and pol necessary for packaging; (C) an envelope plasmid that comprises a gene encoding VSV-G; and (D) a rev expression plasmid that comprises rev.

9. The method for producing a lentiviral vector according to Claim 6, wherein the plurality of plasmids constituting a packaging mix are composed of 3 plasmids: (A) a lentiviral vector plasmid that comprises at least LTRs (5' LTR and 3' LTR), a packaging signal (Ψ), and a target transgene; (B) a packaging plasmid that comprises gag and pol necessary for packaging; and (C) a plasmid that comprises an envelope expression unit comprising a gene encoding VSV-G and a rev expression unit comprising rev.

10. The method for producing a lentiviral vector according to Claim 8 or 9, wherein 5' LTR and 3' LTR in the lentiviral vector plasmid are modified.

11. The method for producing a lentiviral vector according to any one of Claims 3 to 10, wherein Tax is not incorporated into the lentiviral vector.

12. A kit for producing a lentiviral vector in 293T cells comprising: a packaging mix comprising a plurality of plasmids that comprise genes encoding proteins essential for lentiviral particle formation and a plasmid that comprises a gene transcribed into RNA to be incorporated into lentiviral particles containing a transgene to be expressed in target cells; and an expression vector comprising a gene encoding a factor activating a promoter.

13. The kit for producing a lentiviral vector according to Claim 12, wherein the factor activating a promoter is a factor activating a CMV promoter.

14. The kit according to Claim 12 or 13, wherein the factor activating a promoter is one or more factors selected from the group consisting of HTLV-1 Tax, HIV-1 Tat, NF-κB RelA, AP-1, and CREB/ATF.

15. The kit for producing a lentiviral vector in 293T cells according to any one of Claims 12 to 14, which comprises: a packaging mix comprising a plurality of plasmids that comprise genes encoding proteins essential for lentiviral particle formation and a plasmid that comprises a gene transcribed into RNA to be incorporated into lentiviral particles containing a transgene to be expressed in target cells; and an expression vector comprising a gene encoding HTLV-1 Tax or NF-κB RelA.

16. The kit for producing a lentiviral vector in 293T cells according to any one of Claims 12 to 14, which comprises: a packaging mix comprising a plurality of plasmids that comprise genes encoding proteins essential for lentiviral particle formation and a plasmid that comprises a gene transcribed into RNA to be incorporated into lentiviral particles containing a transgene to be expressed in target cells; and an expression vector comprising genes encoding 2 types of factors selected from the group consisting of HTLV-1 Tax, HIV-1 Tat, and NF-κB RelA or 3 types of factors HTLV-1 Tax, HIV-1 Tat, and NF-κB RelA.

17. The kit for producing a lentiviral vector according to Claim 16, wherein the genes encoding 2 types of factors selected from the group consisting of HTLV-1 Tax, HIV-1 Tat, and NF-κB RelA and those 3 types of factors HTLV-1 Tax, HIV-1 Tat, and NF-κB RelA are located on independent plasmid vectors for expression.

18. The kit for producing a lentiviral vector according to any one of Claims 15 to 17, wherein the plurality of plasmids constituting a packaging mix are composed of a lentiviral vector plasmid that comprises at least LTRs (5' LTR and 3' LTR), a packaging signal (Ψ), and a target transgene and a plurality of plasmids that comprise gag, pol, rev, and env independently of each other.

19. The kit for producing a lentiviral vector according to Claim 18, wherein the plurality of plasmids constituting a packaging mix are composed of 3 plasmids: (1) a lentiviral vector plasmid that comprises at least LTRs (5' LTR and 3' LTR), a packaging signal (Ψ), and a target transgene; (2) a packaging plasmid that comprises gag and pol necessary for packaging and may comprise rev and tat regulatory genes; and (3) an envelope plasmid that comprises a gene encoding VSV-G.

20. The kit for producing a lentiviral vector according to Claim 18, wherein the plurality of plasmids constituting a packaging mix are composed of 4 plasmids: (A) a lentiviral vector plasmid that comprises at least LTRs (5' LTR and 3' LTR), a packaging signal (Ψ), and a target transgene; (B) a packaging plasmid that comprises gag and pol necessary for packaging; (C) an envelope plasmid that comprises a gene encoding VSV-G; and (D) a rev expression plasmid that comprises rev.

21. The kit for producing a lentiviral vector according to Claim 18, wherein the plurality of plasmids constituting a packaging mix are composed of 3 plasmids: (A) a lentiviral vector plasmid that comprises at least LTRs (5' LTR and 3' LTR), a packaging signal (Ψ), and a target transgene; (B) a packaging plasmid that comprises gag and pol necessary for packaging; and (C) a plasmid that comprises an envelope expression unit comprising a gene encoding VSV-G and a rev expression unit comprising rev.

22. The kit for producing a lentiviral vector according to Claim 20 or 21, wherein 5' LTR and 3' LTR in the lentiviral vector plasmid are modified.

23. The 293T cell comprising the plasmid and the vector included in the kit according to any one of Claims 15 to 22.
